# EUROPEAN PATENT SPECIFICATION

(11) **EP 4 648 730 B1**
(45) Date of publication and mention of the grant of the patent: **09.09.2026**
(21) Application number: 23837945.7
(22) Date of filing: 22.12.2023
(51) Int. Cl.: A61F 13/49, A61F 13/505, A61F 13/53

(54) **DIAPER ARTICLE WITH MULTIPLE ABSORBENT BODIES HAVING LEG CUFFS**
WINDELARTIKEL MIT MEHREREN SAUGFÄHIGEN KÖRPERN MIT BEINABSCHLÜSSEN
COUCHE À CORPS ABSORBANTS MULTIPLES AVEC MANCHETTE DE JAMBE

(30) Priority: 09.01.2023 WO PCT/CN2023/071285; 09.01.2023 WO PCT/CN2023/071286; 09.08.2023 WO PCT/CN2023/111971
(43) Date of publication of application: 19.11.2025
(73) Proprietor: The Procter & Gamble Company, Cincinnati, OH 45202 (US)
(72) Inventor: TONG, Ling, Beijing 101312 (CN); DAI, Wei, Beijing 101312 (CN); PENG, Lishuang, Beijing 101312 (CN); ZHANG, Shouhai, Beijing 101312 (CN)
(74) Representative: P&G Patent Germany
(86) International application number: PCT/CN2023/141074
(87) International publication number: WO 2024/149049

(56) References cited:
- CN-A- 1 151 688
- US-A- 4 813 950
- US-A1- 2017 239 099
- US-B2- 10 159 609

## Description

### FIELD OF THE INVENTION

This invention relates to diaper articles having multiple layers of absorbent bodies enabling a second usage of the absorbent article after removal of one of the absorbent bodies.

### BACKGROUND OF THE INVENTION

Diaper articles for personal hygiene, such as disposable taped diapers, disposable pants, and adult incontinence undergarments, are designed to absorb and contain various body exudates, including urine, menses, and fecal matter. Diaper articles having multiple absorbent bodies which enables a second usage after the first soiling are desired. Such diaper article requires having multiple absorbent bodies, wherein the first absorbent body may be removed after the first soiling, and thereby exposing another absorbent body. Such diaper articles having multiple absorbent bodies may provide convenience by enabling removing the soiled absorbent body without complete removal of garments. Such convenience may be particularly desired during outing from the home, or during cold seasons. Diaper articles having multiple absorbent bodies may also provide conservation of material.

Diaper articles having multiple absorbent bodies have been known in the art, such as in US5613959, US2017/0216111A, US2017/0216106A, and US2017/0239099A. While such diaper articles have been known, they may require improvements for actual usage.

For example, those known in the art may not have considerations for delivering sufficient containment, leakage prevention, or wear comfort before and after removal of the first absorbent body.

Based on the foregoing, there is a need for a diaper article having multiple layers of absorbent bodies maintaining the performance of containment and wear comfort for the first usage as well as the second usage. There is also a need for a diaper article which addresses the concerns of consumers who are conscious about sustainable usage of material.

### SUMMARY OF THE INVENTION

The present invention is directed to a pant type absorbent article comprising an absorbent assembly and an elastic belt, the absorbent assembly comprising:
1) a first absorbent body comprising:
   a) a first water permeable topsheet;
   b) a first water impermeable backsheet;
   c) a first absorbent core disposed between the first topsheet and the first backsheet;
   d) a first outer cover disposed on the garment facing side of the first backsheet;
   e) a pair of first backsheet fold overs extending from the transverse ends of the first backsheet and folded to the wearer facing side;
   f) a pair of first outer cover fold overs extending from the transverse ends of the first outer cover and folded to the wearer facing side to sandwich the first backsheet fold over, the transverse end of the first outer cover fold over attached to the wearer facing side of the first topsheet, wherein the first backsheet fold over and the first outer cover fold over form a pair of first cuffs, each first cuff comprising a longitudinally extending base and a longitudinally extending free edge; and
   g) a first cuff elastic element disposed at the free edge of each first cuff;
2) a second absorbent body disposed on the garment facing side of the first absorbent body, comprising:
   a) a second water permeable topsheet; and
   b) a second absorbent core;
3) a chassis disposed on the garment facing side of the second absorbent body, the chassis comprising:
   a) a water impermeable chassis backsheet having a greater transverse dimension than the first absorbent body;
   b) a pair of chassis cuffs formed by a cuff material and disposed on the chassis backsheet, the chassis cuffs each comprising a longitudinally extending base and a longitudinally extending free edge; wherein the transverse dimension of the spacing between the bases of the pair of chassis cuffs TC2 are greater than the transverse dimension of the spacing between the bases of the pair of first cuffs TC1;
   c) a chassis cuff elastic element disposed at the free edge of each chassis cuff;
   wherein the absorbent article exhibits a Whole Longitudinal Leg Tensile and a Whole Transverse Leg Tensile, the absorbent article devoid of the first absorbent body exhibits a Removed Longitudinal Leg Tensile and a Removed Transverse Leg Tensile, wherein the Removed Longitudinal Leg Tensile is at least about 50% of the Whole Longitudinal Leg Tensile, and the Removed Transverse Leg Tensile is at least about 50% of the Whole Transverse Leg Tensile, according to measurements herein.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1A - 1B are schematic perspective views of an embodiment of the diaper article of the present invention showing how the first absorbent body is removed from the remainder of the diaper article.
Figure 2A is a schematic plan view of an embodiment of the absorbent assembly of the present invention showing the wearer-facing side wherein above TX the cuff elements are removed, wherein right to LX and below TX only elements of the first absorbent body are shown, and wherein left to LX and below TX only elements of the second absorbent body and chassis are shown.
Figure 2B is a schematic plan view of another embodiment of the absorbent assembly of the present invention showing the wearer-facing side wherein above TX the cuff elements are removed, wherein right to LX and below TX only elements of the first absorbent body are shown, and wherein left to LX and below TX only elements of the second absorbent body and chassis are shown.
Figure 3A is an embodiment of a transverse schematic cross section view of Figure 2A taken along line 3-3.
Figure 3B is another embodiment of a transverse schematic cross section view of Figure 2B taken along line 3-3.
Figure 4 is a schematic plan view of an embodiment of a pant diaper article of the present invention with the seams unjoined and in a flat uncontracted condition showing the garment facing surface.
Figure 5 is a schematic view of an example of a hanger-type sample holding fixture according to the "Leg Opening Tensile Measurement" herein.

### DEFINITIONS

As used herein, the following terms shall have the meaning specified thereafter:
"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", prefastened diapers", "pull-on diapers", "training pants" and "diaper-pants".

"Longitudinal" "Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Proximal" and "distal" refer respectively to the position closer or farther relative to the longitudinal center of the article.

"Wearer-facing" and "garment-facing" refer respectively to the relative location of an element or a surface of an element or group of elements. "Wearer-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable diaper article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m² or gsm).

"Water-permeable" and "water-impermeable" refer to the penetrability of materials in the context of the intended usage of disposable diaper articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Dimension", "Length", "Width", "Pitch", "Diameter", "Aspect Ratio", "Angle", and "Area" of the article are all measured in a state wherein the article is extended to the Full Stretch Circumference W1 according to the "Whole Article Force Measurement" herein, and utilizing a ruler or a loupe, unless specified otherwise.

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to 0 ± 2, and a b* value equal to 0 ± 2 according to the CIE L* a* b* color system.

### DETAILED DESCRIPTION OF THE INVENTION

### Diaper Article and Absorbent Assembly

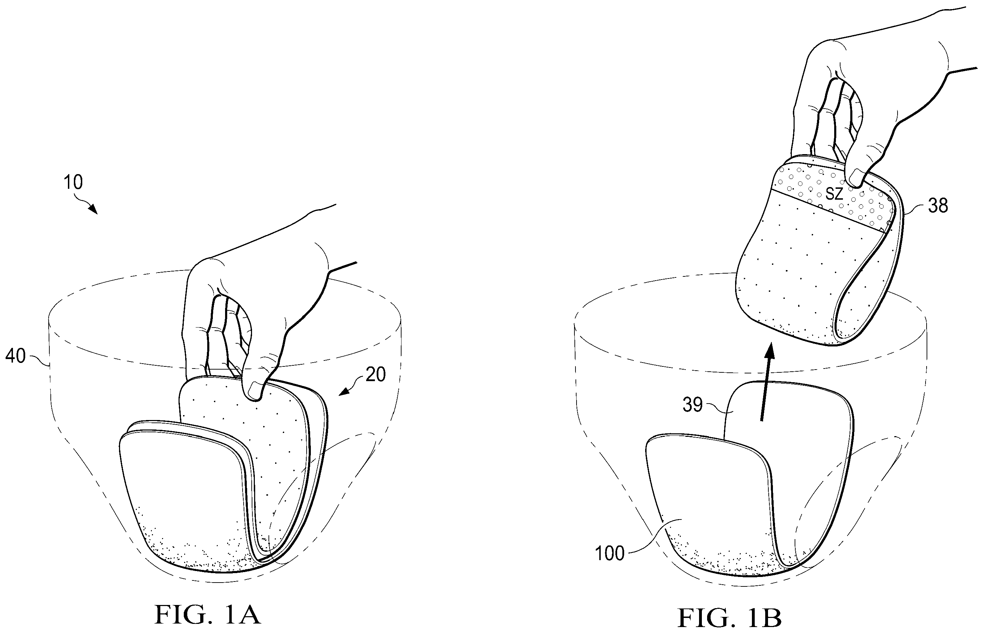

Figures 1A-1B are perspective views of an embodiment of the diaper article (10) of the present invention of the pant type comprising an absorbent assembly (20) and an application means (40). The absorbent assembly (20) comprises a first absorbent body (38), a second absorbent body (39) disposed on the garment facing side of the first absorbent body, and a chassis (100) disposed on the garment facing side of the second absorbent core, wherein the first absorbent body (38) and the second absorbent body (39) is bonded with a Temporary Bond (TB) which enables removing the first absorbent body (38) from the remainder of the diaper article (10). The diaper article (10) of the present invention has multiple layers of absorbent bodies (38, 39), enabling usage of the diaper article (10) more than once. Accordingly, the chassis (100) and the application means (40) may be used more than once, thus contributing to sustainable usage of material. Further, the first absorbent body (38) may be removed from the remainder of the diaper article (10) without completely removing the diaper article (10) from the wearer, facilitating easy diaper change.

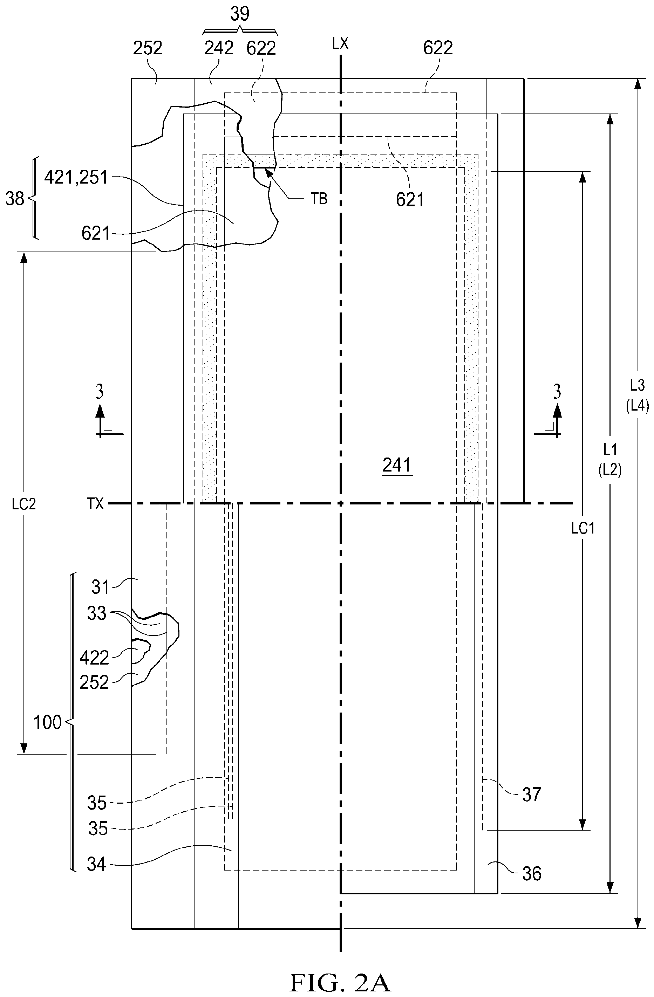

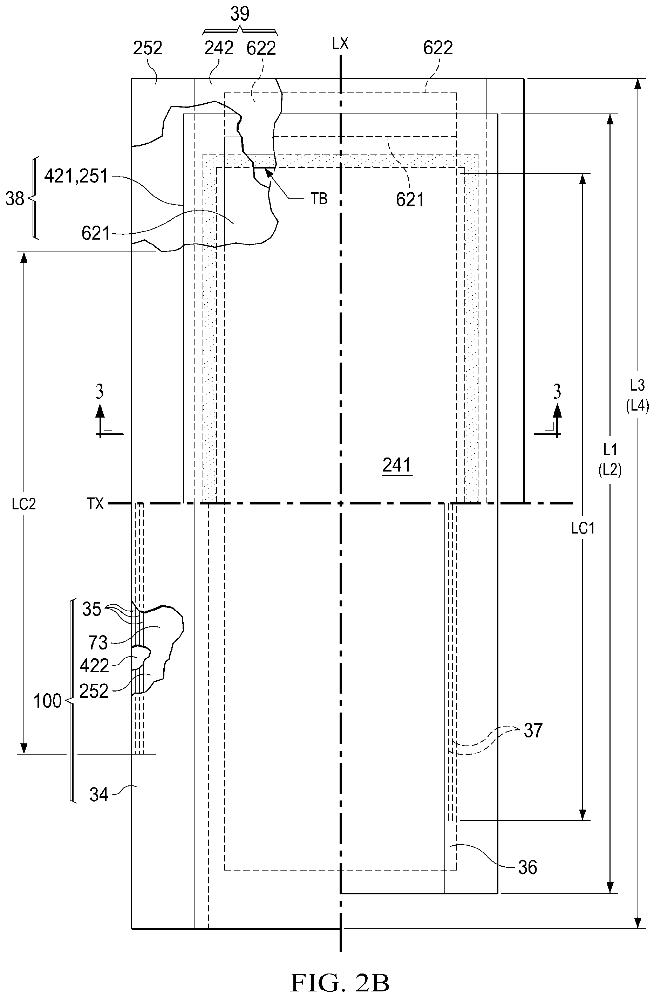

Referring to Figures 2A-2B, the absorbent assembly (20) has a longitudinal centerline LX which also serves as the longitudinal axis, and a transverse centerline TX which also serves as the transverse axis. Figures 2A-2B are schematic plan views of embodiments of the absorbent assembly of the present invention showing the front side on the top and back side on the bottom, wherein above TX, showing the wearer-facing side, wherein the cuff material (110), the cuff elastic elements, and any parts of the first absorbent body extending outwardly from the transverse edges of the first topsheet are removed. Namely, above TX, the cuff material (110) and the cuff elastic elements (33, 35, 37) are removed. Hence, the entirety of the first topsheet (241) and any elements extending beyond the dimension of the first topsheet (241) are shown. Towards the upper left side of Figures 2A-2B, the elements of the first absorbent body (38) beneath the first topsheet (241) are exposed and shown. Still further, the elements of the second absorbent body (39) beneath the first outer cover (421) are exposed and shown. Figures 2A and 2B below TX are shown in a state wherein any elastic cuff elements (33, 35) are fully stretched. Towards the lower right of Figures 2A and 2B, right to LX and below TX, only elements of the first absorbent body are shown. Towards the lower left of Figures 2A and 2B, left to LX and below TX, only elements of the second absorbent body and chassis are shown. Further, in this lower left side of this region, the elements of the chassis (100) beneath the nonwoven forming the chassis outer cuff (34) are exposed and shown.

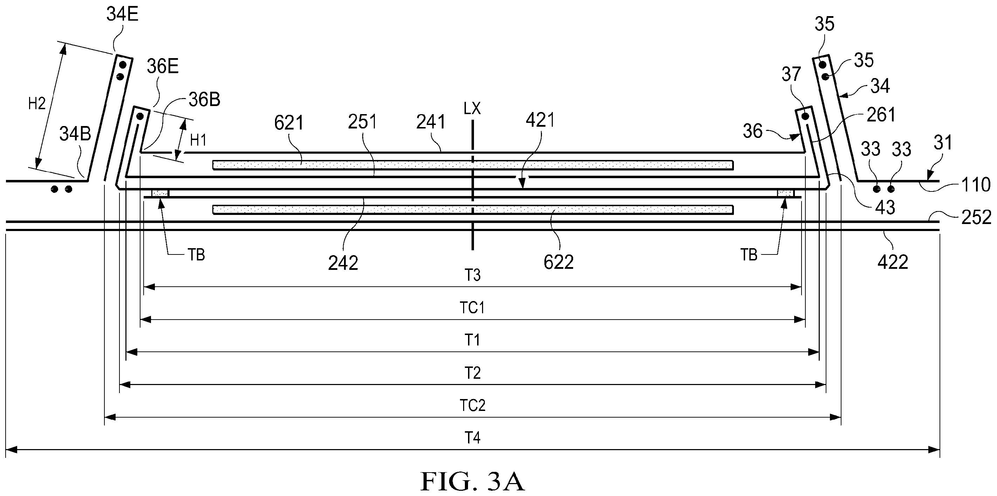

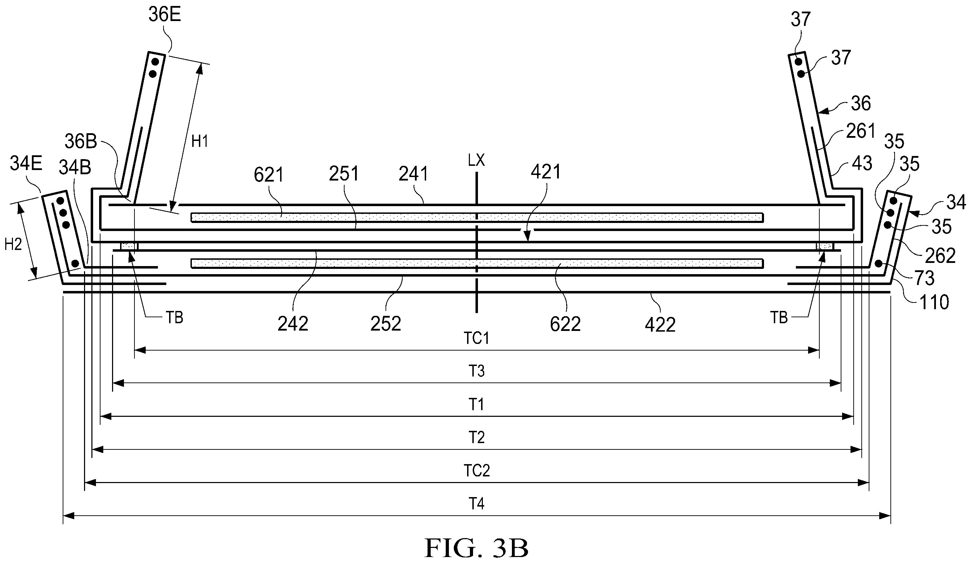

Referring to Figures 2A, 2B, 3A and 3B, the first absorbent body (38) and the remainder of the diaper article (39, 100) each carry essential components in order to provide the function as an absorbent diaper article (10). Specifically, the first absorbent body (38) comprises a first water permeable topsheet (241), a first water impermeable backsheet (251), a first absorbent core (621) disposed between the first topsheet and the first backsheet, and a first outer cover (421). The first outer cover (421) serves as the interface between the second absorbent body (39) on which the Temporary Bond (TB) is provided. The second absorbent body (39) comprises a second water permeable topsheet (242) and a second absorbent core (622). The second topsheet (242) serves as the interface between the first absorbent body (38) on which the Temporary Bond (TB) is provided. A chassis (100) is disposed on the garment facing side of the second absorbent core, which chassis (100) comprises a water impermeable chassis backsheet (252) which may serve as the barrier to prevent leakage of exudates absorbed by and contained in any absorbent body within the absorbent assembly (20). When there are only first and second absorbent bodies (38, 39), the second absorbent body (39) may be devoid of a second backsheet.

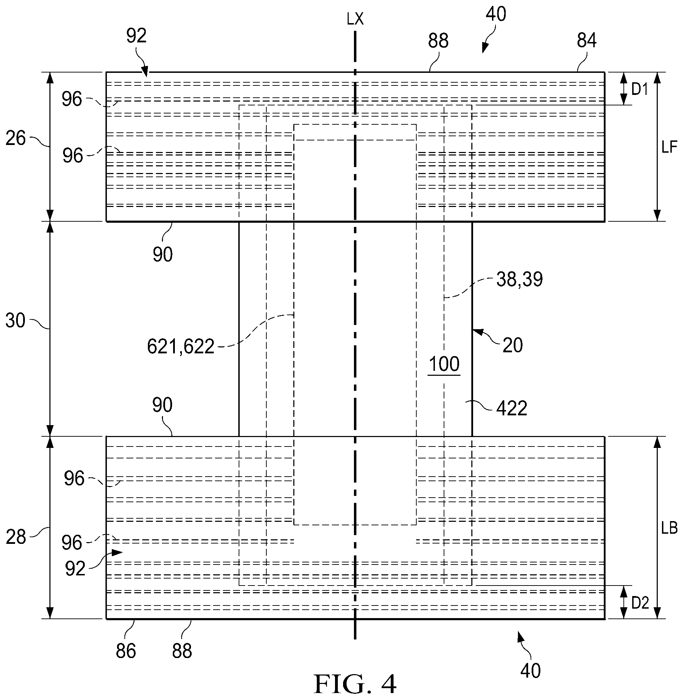

The absorbent assembly (20) of the present invention is assembled together with an elastic belt (40) to form a pant type absorbent article. Referring to Figure 4, the diaper article (10) may comprise an elastic belt (40), wherein the elastic belt (40) may be formed by nonwoven materials and/or woven materials combined with elastic materials such as elastic bodies and films, which are discussed in further detail below. The pant diaper article (10) of the present invention enables more than one usage, while also has an appropriate thickness and thereby are comfortable to wear. The diaper article (10) of the present invention may have a Folded Thickness of no greater than about 12.5mm according to the measurement herein. By providing the diaper article within such Folded Thickness, the article provides the aesthetic and tactile senses similar to that of a diaper article with a single absorbent body.

### Topsheet, Backsheet, and Outer Cover

Referring to Figures 2A, 2B, 3A and 3B, the absorbent bodies (38, 39) of the present invention each comprise a water permeable topsheet (241, 242) that may be positioned at least in partial contact or close proximity to a wearer. The same or different material may be used for the first and second absorbent bodies (38, 39). Suitable topsheets (241, 242) may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet (241, 242) is generally supple, soft feeling, and non-irritating to a wearer's skin. The topsheet (241, 242) is liquid permeable, permitting bodily fluids to readily penetrate through the thickness of the topsheet (241, 242). Exemplary topsheets (241, 242) suitable herein include those available from Xiamen Yanjan New Material Co. Ltd made of carded nonwoven substrate comprising PE/PET bi-component fibers, Fibertex NiLai, Malaysia with tradename H30501221 or FQN Hazlet NJ with tradename SB1206169. Any portion of the topsheet (241, 242) may be coated with a lotion or skin care composition as is known in the art. Examples of suitable lotions include those described in U.S. Patent Nos. 5,607,760; 5,609,587; 5,635,191; and 5,643,588.

The first absorbent body (38) and the chassis (100) of the present invention each comprise a water impermeable backsheet (251, 252) which is designed to prevent the exudates absorbed by and contained within the absorbent core (621, 622) from soiling articles that may contact the diaper article (10), such as bed sheets and undergarments. The same or different material may be used for the first absorbent body (38) and the chassis (100). The backsheets (251, 252) may be positioned such that it extends beyond the absorbent core (621, 622) disposed on the wearer-facing side of the particular backsheet (251, 252) in both the longitudinal direction and the transverse direction. Suitable backsheet (251, 252) materials include films such as those manufactured by Plaster Argentina with tradename PLBA NBBS 10-12GSM PR V1. Other suitable backsheet (251, 252) materials may include breathable materials that permit vapors to escape from the diaper article (10) while still preventing exudates from passing through the backsheet (251, 252). Exemplary breathable materials may include materials such as woven webs, nonwoven webs, composite materials such as film-coated nonwoven webs, and microporous films such as manufactured by Daika Japan with tradename MPF DKH-180 15G V7 and manufactured by Berry Nashville, TN with trademark BR-137P V13. Such breathable composite materials are described in greater detail in PCT Application No. WO 95/16746 and US5,865,823. Other breathable backsheets including nonwoven webs and apertured formed films are described in US5,571,096. An exemplary, suitable backsheet is disclosed in US6,107,537. Other suitable materials and/or manufacturing techniques may be used to provide a suitable backsheet including, but not limited to, surface treatments, particular film selections and processing, particular filament selections and processing, etc.

The first absorbent body (38) comprises a first outer cover (421) which serves as the interface between the second absorbent body (39). The first outer cover (421) may be made of a soft, non-woven material. The first outer cover (421) and the first backsheet (251) may be joined together by adhesive or any other suitable material or method. An optional chassis outer cover (422) may also be provided on the garment-facing side of the chassis backsheet (252) for providing the diaper article in a finished appearance. Exemplary outer covers (421, 422) suitable herein include those available from Guanhe Hygiene Products Co., Ltd with tradename S31921A made of spunbond nonwoven substrate comprising PP fiber, and Fibertex NiLai Malaysia with tradename A10160EJ - MALAYSIA and available from FQN Hazlet NJ with tradename SM1104174.

### Absorbent Core

Referring to Figures 2A, 2B, 3A and 3B, the absorbent bodies (38, 39) of the present invention each comprise an absorbent core (621, 622) for absorbing and containing body exudates disposed on the wearer facing side. The absorbent cores (621, 622) may include an absorbent layer and an acquisition system. The absorbent layer is the region wherein absorbent materials having a high retention capacity, such as superabsorbent polymers, are present. The absorbent layer may be substantially cellulose free. Alternatively, the absorbent layer may contain cellulose. There may be an absorbent layer mainly comprising cellulose, and another absorbent layer mainly comprising superabsorbent polymers.

Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multi constituent fibers such as side by side, core/sheath or island in the sea type fibers. Such synthetic fibers may be formed via a spunbonding process or a meltblowing process. Some portions of the absorbent layers may be configured to have substantially no absorbent material to form a channel or a plurality of channels. Channels may be useful for allowing the absorbent core (621, 622) to bend upon swelling with fluids, such that the crotch region conforms to the wearer's body after swelling and prevent sagging of the diaper article (10). The absorbent layers may be disposed in plurality in the absorbent core (621, 622).

Alternatively, the absorbent core (621, 622) may comprise a high loft material encompassing superabsorbent polymers. The term "high loft" refers to low density bulky fabrics, as compared to flat, paper-like fabrics. High loft webs are characterized by a relatively high porosity. This means that there is a relatively high amount of void space in which superabsorbent polymer particles can be distributed. The high loft material (without the superabsorbent particles) may have a density at a pressure of 4.14kPa (0.6 psi) below 0.20 g/cm³, in particular ranging from 0.05 g/cm³ to 0.15 g/cm³. The high loft layer (without the superabsorbent particles) may have a density at a pressure of 2.07 kPa (0.3 psi) below 0.20 g/cm³, in particular ranging from 0.02 g/cm³ to 0.15 g/cm³. The high loft layer (without the superabsorbent particles) may have a density at a pressure of 0.83 kPa (0.12 psi) below 0.15 g/cm³, in particular ranging from 0.01 g/cm³ to 0.15 g/cm³, and a basis weight of from 15 to 500gsm, preferably 30~200gsm, such as those described in US 2021/0361497 Al. The absorbent core (621, 622) comprising high loft material encompassing superabsorbent polymers may also contain channels.

The absorbent cores (621, 622) may also contain an acquisition system for facilitating the acquisition and the distribution of body exudates, and may be placed between the topsheet (241, 242) and the absorbent layer. The function of the acquisition system is to rapidly acquire the fluid from the topsheet (241, 242) away from the wearer-facing side and/or to distribute over a larger area so it is more efficiently absorbed by the absorbent core. The acquisition system may include cellulosic fibers. Channels may also be formed in the acquisition system, and may be configured to at least partly match the channels of the absorbent layer in the thickness direction. It is also possible that such another liquid management layer may be placed between the backsheet (251, 252) and the absorbent layer. The liquid management layer may be a spunlace nonwoven comprising viscose, PET, CoPET/PET fibers, and combinations thereof.

### Leg Cuffs

Referring to Figures 3A and 3B, the absorbent bodies (38, 39) of the present invention each comprise leg cuffs (31, 34, 36) for gasketing the transverse edges of the absorbent article (10) against the wearer's thighs and prevent bodily exudates from leaking from the leg openings. State of the art absorbent articles typically have a dual leg cuff gasketing system for providing leakage prevention from the leg opening, while also providing a soft touch to the wearer around the thighs. Simply relying on such dual leg cuff gasketing system on the chassis (100) is not enough for leakage prevention of the multi absorbent body article of the present invention. On the other hand, simply applying such dual leg cuff gasketing system to each of the absorbent bodies (38, 39) or each of the first absorbent body (38) and chassis (100) of the present invention would render the absorbent article (10) overly bulky around the wearer's thighs with unnecessary number of layers of material in this region. Such bulkiness or concentrated excess number of layers of materials may risk red marking against the wearer, or even affect leakage prevention function. There is required special configurations for the leg cuffs of the absorbent article of the present invention in order to provide side leakage function in both the first and second usage, while also maintaining wear comfort.

Still referring to Figures 3A and 3B, the first absorbent body (38) of the present invention comprises a pair of first backsheet fold overs (261) extending from the transverse ends of the first backsheet (251) and folded to the wearer facing side; a pair of first outer cover fold overs (43) extending from the transverse ends of the first outer cover (421) and folded to the wearer facing side to sandwich the first backsheet fold over (261), the transverse end of the first outer cover fold over (43) attached to the wearer facing side of the first topsheet (241), wherein the first backsheet fold over (261) and the first outer cover fold over (43) form a pair of first cuffs (36), each first cuff (36) comprising a longitudinally extending base (36B) and a longitudinally extending free edge (36E); a first cuff elastic element (37) disposed at the free edge of each first cuff (36). Further, the chassis (100) of the present invention comprises a pair of chassis cuffs (34) formed by a cuff material (110) and disposed on the chassis backsheet (252), the chassis cuffs (34) each comprising a longitudinally extending base (34B) and a longitudinally extending free edge (34E); wherein the transverse dimension TC2 of the spacing between the bases of the pair of chassis cuffs (34B) are greater than the transverse dimension TC1 of the spacing between the bases of the pair of first cuffs (36B); and a chassis cuff elastic element (35) is disposed at the free edge of each chassis cuff (34). Still further, the absorbent article of the present invention (10) exhibits a Whole Longitudinal Leg Tensile and a Whole Transverse Leg Tensile, the absorbent article devoid of the first absorbent body exhibits a Removed Longitudinal Leg Tensile and a Removed Transverse Leg Tensile, wherein the Removed Longitudinal Leg Tensile is at least about 50% of the Whole Longitudinal Leg Tensile, and the Removed Transverse Leg Tensile is at least about 50% of the Whole Transverse Leg Tensile, according to measurements herein. By "Whole" Longitudinal/Transverse Leg Tensile, what is meant is the unloading force (N) of the leg opening when the absorbent article is in first usage. By "Removed" Longitudinal/Transverse Leg Tensile, what is meant is the unloading force (N) of the leg opening when the absorbent article is in second usage after removal of the first absorbent body (38). By Whole/Removed "Longitudinal" Leg Tensile, what is meant is the unloading force of the leg opening in the longitudinal direction of the article. By Whole/Removed "Transverse" Leg Tensile, what is meant is the unloading force of the leg opening in the transverse direction of the article. By providing the Removed Longitudinal Leg Tensile to be at least about 50% of Whole Longitudinal Leg Tensile, and the Removed Transverse Leg Tensile to be at least about 50% of the Whole Transverse Leg Tensile, this provides balanced leg gasketing against the wearer in both the first usage and the second usage.

Specifically, the difference between the Removed Longitudinal Leg Tensile and the Whole Longitudinal Leg Tensile may be less than about 1.5N, and the difference between the Removed Transverse Leg Tensile and the Whole Transverse Leg Tensile may be less than about 1.5N. The Whole Longitudinal Leg Tensile may be from about 1N to about 3.5N, or from about 1.3N to about 3.3N, and the Whole Transverse Leg Tensile may be from about 0.5N to about 2N, or from about 0.7N to about 1.3N. The Removed Longitudinal Leg Tensile may be from about 0.5N to about 3N, or from about 0.8N to about 2.3N, and the Removed Transverse Leg Tensile may be from about 0.2N to about 1N, or from about 0.3N to about 0.8N.

The aforementioned tensile profiles may be actualized by providing less than a dual leg cuff gasketing system for one or both of the first absorbent body (38) and the chassis (100) of the present invention. Further, when the first cuff elastic element (37) and the chassis cuff elastic element (35) are a plurality of elastic strands, respectively, the aforementioned tensile profiles may be created by adjusting one or more of the: density (dtex) of the elastic strands, elongation of the elastic strands, and number of elastic strands. There may be more number of chassis cuff elastic strands (35) than the first cuff elastic strands (37).

Referring to Figure 3A, when the first cuff (36) has a first cuff height H1 which is the transverse dimension between the base (36B) and the free edge (36E); while the chassis cuff (34) has a chassis cuff height H2 which is the transverse dimension between the base (34B) and the free edge (34E), H2 may be greater than H1, wherein the chassis further comprises a pair of outer cuffs (31) disposed transversely outboard of the pair of chassis cuffs (34), respectively, each outer cuff (31) formed by the chassis backsheet (252), the cuff material (110), and an outer cuff elastic element (33) sandwiched therebetween. In this embodiment, there are 3 cuff elements in the first usage, and 2 cuff elements in the second usage, wherein the 2 cuff elements on the chassis (31, 34) play a primary leg gasketing function, while the first cuff (36) provides an auxiliary function while the first absorbent body (38) is present. The chassis cuff height H2 is provided greater than the first cuff height H1 for sustained fit before and after removal of the first absorbent body (38). In this embodiment, the first cuff (36) provides leakage prevention from the first absorbent body (38) to the second absorbent body (39). In this embodiment, the chassis (100) may further comprise a chassis outer cover (422) on the garment facing side of the chassis backsheet (252), wherein the transverse dimension of the chassis backsheet (252) matches that of the chassis outer cover (422). This provides a finished appearance for the absorbent article (10).

Alternatively, referring to Figure 3B, an edge elastic element (73) may be disposed adjacent each base of the chassis cuffs (34B), wherein when the first cuff (36) has a first cuff height H1 and the chassis cuff (34) has a chassis cuff height, H2 may be smaller than H1. In this embodiment, the chassis cuff (34) plays a primary leg gasketing function in the first usage, and the edge elastic element (73) provides structure to the chassis cuff (34). The chassis cuff height H2 is provided smaller than the first cuff height H1 to provide better leakage prevention from the first to second absorbent body, while also providing improved appearance as an absorbent article as a whole, as having less bulkiness around the cuff area. In this embodiment, the chassis (100) may further comprises a pair of chassis backsheet fold overs (262) extending from the transverse ends of the chassis backsheet (252) and folded to the wearer facing side, wherein the cuff material (110) sandwiches the chassis backsheet fold over (262), the plurality of chassis cuff elastic strands (33), and edge elastic elements (73) to form the chassis cuffs (34) for leakage prevention, and to provide structure to the cuff area on the chassis (100). Also in this embodiment, the chassis (100) may further comprise a chassis outer cover (422) on the garment facing side of the chassis backsheet (252), wherein the base of the chassis cuff (34) is disposed adjacent the transverse ends of the chassis outer cover (422). This provides a finished appearance for the absorbent article (10).

### Dimensions

Referring to Figures 3A and 3B, the components for forming the absorbent assembly (20) may have a particular relationship with one another for ensuring that the second absorbent body (39) remains intact during the first usage, while maintaining wear comfort and enabling smooth removal of the first absorbent body (38) after usage. Hereinafter, it is defined that the first backsheet (251) has a transverse dimension T1 and a longitudinal dimension L1, the first outer cover (421) has a transverse dimension T2 and a longitudinal dimension L2, the second topsheet (242) has a transverse dimension T3 and a longitudinal dimension L3, and the chassis backsheet (252) has a transverse dimension T4 and a longitudinal dimension L4. Further, as described above, the transverse dimension between the bases of the pair of first cuffs (36B) is TC1, and the transverse dimension between the bases of the pair of chassis cuffs (34B) is TC2.

The Temporary Bond (TB) for bonding the first and second absorbent bodies (38, 39) is provided between the first outer cover (421) and the second topsheet (242) such that the first absorbent body (38) may be removed from the remainder of the diaper article (10) after first usage. In order not to soil the second absorbent body (39) during first usage, T1 may be provided equal to or greater than T3, preferably T1 may be provided greater than T3 by from about 5mm to about 15mm. Further, T2 may be provided equal to or greater than T3 for the same purpose, preferably T2 may be provided greater than T3 by from about 5mm to about 45mm.

Referring to Figures 3A-3B, the first absorbent body (38) may further comprise a first outer cover fold over (43) connected to the first outer cover (421), wherein the transverse edges of the first backsheet (251) is sandwiched by the first outer cover (421) and the first outer cover fold over (43). By providing the elements of the first absorbent body (38) in such configuration, there is provided improved protection along the transverse edges of the absorbent assembly (20), and visual exposure of the first backsheet (251) to the user may be avoided. Visual exposure of the backsheet (251, 252) is typically avoided for the purpose of providing the article a finished appearance, and also for avoiding exposure of color of exudates after soiling of the absorbent assembly (20). For purpose of determination of dimensions T1, T2, and T4, respectively, only the first backsheet (251), the first outer cover (421), and the chassis backsheet (252) are concerned, and the dimensions of the first backsheet fold over (261), the first outer cover fold over (43), and the chassis backsheet fold over (262) are not counted.

Referring to Figures 2A-2B, L3 may be provided greater than L1, preferably L3 may be provided greater than L1 by from about 20mm to about 60mm. Namely, the front end and/or the back end of the second topsheet (242) may be extended beyond the first backsheet (251) as well as the first outer cover (421). By providing the first absorbent body (38) and the second absorbent body (39) in such configuration, the user may conveniently remove the first absorbent body (38) from the remainder of the absorbent assembly by inserting fingers between the first outer cover (421) and the second topsheet (242). The chassis backsheet (252) provides barrier function for the entire diaper article (10). In order to provide such function, T4 may be greater than T1, preferably T4 may be provided greater than T1 by from about 40mm to about 60mm. T4 may be provided greater than any of T1, T2, or T3. L4 may be greater than L1 or L2. The first absorbent core is provided smaller in dimension than T1, T2, L1, and L2. The second absorbent core is provided smaller in dimension than T3, T4, L3, and L4. For purpose of clarification, in Figures 3A-3B, T2 is shown in greater dimension than T1 for describing how the first outer cover (421) provides a fold over (43) at the left and right folding points, thus adding dimension. In reality, however, the embodiment described in Figures 3A-3B may have T1 and T2 in the same transverse dimension.

Referring to Figures 2A-2B, the absorbent core existing on the garment facing side, namely the second absorbent core (622), may have the distance between the front end of the core and the front end of the absorbent assembly (20) shorter than the distance between the back end of the core and the back end of the absorbent assembly (20). By somewhat shifting the core towards the front than in the longitudinal center, the absorbent core may effectively receive and absorb fluid waste. When the second absorbent core (622) is disposed at such position relative to the absorbent assembly (20), the back end of the first and second absorbent cores (621, 622) may be matched, while the front end of the first absorbent core (621) exists proximal from the front end of the second absorbent core (622). By providing the first and second absorbent cores (621, 622) in such position, the vicinity of the longitudinal edge of the first outer cover (421) on the front side may be kept thin by being devoid of the first absorbent core (621), thus facilitating inserting fingers between the first outer cover (421) and the second topsheet (242) for the removing activity of the first absorbent body (38). Such longitudinal dimension difference of the first and second absorbent cores (621, 622) may be provided on the front side, where there is remained less longitudinal dimension between the front end of the absorbent cores (621, 622) and the absorbent assembly (20).

Referring to Figures 3A and 3B, the transverse dimension of the first backsheet T1 may be the same or greater, preferably greater, than the spacing between the bases of the first cuffs TC1. By providing the first backsheet T1 to have at least the same transverse dimension, the first backsheet (251) serves as a barrier for bodily exudates passing the side edges of the first cuffs (36) beyond TC1. As mentioned above, the first cuffs (36) may include the first backsheet fold over (261) sandwiched between the cuff material (110) which may provide increased protection in this region. The spacing between the bases of the chassis cuffs TC2 is greater than the transverse dimension of the first backsheet T1. The spacing between the base of the chassis cuffs TC2 is also greater than the transverse dimension of the first outer cover T2. By providing the transverse dimension between the chassis cuffs (TC2) greater than T1 as well as T2, namely any element of the first absorbent body (38), this prevents any bodily exudates passing the first absorbent body (38) to be captured by the chassis cuff (34). In one preferred embodiment, by providing the relationship of TC1 < T1 < T2 < TC2, this provides the overall absorbent article to have wearer comfort as well as good gasketing function.

Referring to Figures 2A and 2B, the first cuff elastic element (37) and the chassis cuff elastic element (35) may each have an effective longitudinal elasticity dimension. By effective longitudinal elasticity dimension, what is meant is the longitudinal dimension of a cuff elastic element which is in active elasticity in its fully stretched length. Namely, portions of the cuff elastic element deprived of elasticity by, for example, bonding against the cuff material (110) and/or the first/second topsheet (241/242) are not counted. Tack down bonding of the cuff elastic elements towards the front and back longitudinal ends of a cuff elastic element is a common method used for controlling the length of the effective longitudinal elasticity dimension of a cuff elastic element. The cuff elastic elements (33, 35, 37, 73) of the present invention may be controlled of its effective longitudinal elasticity dimension by this method. In one preferred embodiment, wherein the first cuff elastic element (37) has an effective longitudinal elasticity dimension of LC1 and the chassis cuff elastic element (35) has an effective longitudinal elasticity of LC2, LC1 is greater than LC2. By providing LC1 greater than LC2, the first absorbent body (38) is kept in close proximity to the wearer during first usage, so that leakage from the first absorbent body (38) to the second absorbent body (39) is prevented.

The substrate elements of the absorbent assembly (20) are all depicted in rectangles in Figures 2A and 2B. Alternatively, the 4 edges of the elements of the absorbent assembly (20), and particularly elements of the first absorbent body (38), may be rounded off such as in Figures 1A-1B. Such rounding off may be beneficial for avoiding the first absorbent body (38) having sharp edges, which sharp edges may otherwise be well observed during the removing action by the user. For purpose of determination of dimensions T1, T2, T3, T4, TC1, TC2, L1, L2, L3, L4, LC1 and LC2, the maximum transverse and/or longitudinal dimensions of each element is measured.

### Temporary Bond

The absorbent assembly (20) of the present invention has the first outer cover (421) and the second topsheet (242) bonded with a Temporary Bond (TB) for enabling removal of the first absorbent body (38) from the remainder of the diaper article (10), after the first use. The Temporary Bond (TB) is provided between two nonwoven layers, the first outer cover (421) and the second topsheet (242) with an appropriate peeling strength. The Temporary Bond (TB) may be provided by any of adhesive, heat energy, ultrasonic energy, or combinations thereof. The Temporary Bond (TB) may have a Peak Force of about 16N or lower, or a Peak Force of from about 4N to about 15N, or a Peak Force of from about 4N to about 10N, or a Peak Force of from about 6N to about 15N, or a Peak Force of from about 10N to about 15N, and an Average Force of about 0.3N or higher, or an Average Force of from about 0.4N to about 15N, or an Average Force of from about 1N to about 15N, or an Average Force of from about 0.4N to about 14N, according to the Peel Strength Measurement herein. The Peel Strength Measurement herein intends to mimick the removing activity of the user when the first absorbent body (38) is removed from the remainder of the diaper article by starting the breaking of the Temporary Bond (TB) from either of the front or back end of the first absorbent body (38) towards the other of the front or back end. The Average Force resembles the force observed during the middle of the removing activity. By having an Average Force of about 0.3N or higher, the Temporary Bond (TB) is strong enough to endure the forces created during the first usage by the wearer. The Peak Force resembles the maximum force experienced during the removing activity. By having a Peak Force of about 16N or lower, the Temporary Bond (TB) is weak enough that the bond may be broken without significant effort and without destroying other components of the diaper article (10).

So long as the above mentioned Peak Force and Average Force according to the Peel Strength Measurement herein are achieved, the first outer cover (421) and the second topsheet (242) may be any nonwoven material suitable for use as a diaper article (10). Exemplary first outer cover (421) materials may be selected from those having a basis weight of from about 6.5gsm to about 25gsm, preferably from about 8gsm to about 20gsm. Exemplary second topsheet (242) materials may be selected from those having a basis weight of from about 11gsm to about 40gsm, preferably from about 15gsm to about 40gsm. The first outer cover (421) may have a lower basis weight than that of the second topsheet (242).

The Temporary Bonds (TB) may be provided at least partially along the transverse edges of the second topsheet (242) and extending in the longitudinal direction. The Temporary Bonds (TB) may be a substantially straight line along the left and right transverse edges of the second topsheet (242). Alternatively, the Temporary Bond (TB) may be a continuous line, such as in oval shape, extending along at least partially the transverse edges of the second topsheet (242). The continuous line may be in the shape of rectangle, or other polygon. There may further be provided an Auxiliary Temporary Bond (ATB) between the first outer cover (421) and the first outer cover (421), wherein the Auxiliary Temporary Bond (ATB) provides the same or lower Peak Force as the Temporary Bond (TB) according to the Peel Strength Measurement herein. The Auxiliary Temporary Bond (ATB) may be disposed between the Temporary Bonds (TB) extending in the transverse direction, wherein the Auxiliary Temporary Bond (ATB) is devoid in the front and back longitudinal edge regions. The Auxiliary Temporary Bond (ATB) may be connected with the left and right Temporary Bonds (TB).

### Elastic belt

Referring to Figure 4, the pant type diaper article (10) of the present invention comprises an elastic belt (40), the elastic belt (40) comprising a front belt (84), a back belt (86), and a pair of side seams (32) sealing the transverse edges of the front belt (84) and the back belt (86). The pant diaper article (10) may be a belt-type pant as in Figure 4 wherein the front belt (84) and the back belt (86) are discontinuous in the longitudinal direction, and assembled with the absorbent assembly (20) wherein the front and back belts (84, 86) are positioned on the garment facing side of the absorbent assembly (20), wherein by the side seam (32) sealing the front and back belts (84, 86) form a ring-like belt. For the belt-type pant, the front belt (84) may be referred to as the front region (26), the back belt (86) may be referred to as the back region (28), and the remainder may be referred to as the crotch region (30). The front and back belts (84, 86) may be rectangular. At least one of the front and back belts (84, 86) may be shaped (not shown). For the belt-type pant, the longitudinal dimension (LB) of the back belt (86) may be greater than that (LF) of the front belt (84), wherein the distal edges (88) of the front belt (84) and the back belt (86) are matched for seaming, thereby leaving the proximal edges (90) of the back belt (86) remaining unsealed. The unsealed proximal edges of the back belt (86) may form a buttock cover. While not shown, the pant elastic article (10) may be a one-piece type wherein the front and back belt (86) are continuous.
The front belt (84) and the back belt (86) may each be formed by a laminate comprising a plurality of elastic bodies (96) running in the transverse direction. At least some of the elastic bodies (96) extend in the transverse direction substantially parallel to each other. The front belt (84) and the back belt (86) may each comprise a laminate, the laminate comprising a plurality of elastic bodies (96) running in the transverse direction, an inner sheet (94), an outer sheet (92), and an outer sheet fold over (93) wherein the outer sheet fold over (93) is an extension of the outer sheet material formed by folding the outer sheet material at the distal edge (88) of the front and back belts; wherein the elastic bodies (96) are sandwiched between two of these sheets. The outer sheet fold over (93) from the front and back belts (84, 86) may sandwich the front and back longitudinal edges of some components of the absorbent assembly (20). The front elastic belt (84) and the back elastic belt (86) may each be made only by elastic bodies (96), the inner sheet (94), the outer sheet (92), and the outer sheet fold over (93). The elastic bodies (96) may be disposed in the same or different denier, interval, and force between the front and back, as well as in different longitudinal positions of the belt.

Still referring to Figure 4, the pant diaper article (10) is formed by the absorbent assembly (20), the front belt (84) and the back belt (86), wherein the position of the absorbent assembly (20) in view of the front belt (84) and back belt (86) are arranged in order to provide good absorbency as well as good wear comfort. When the pant diaper article (10) has the seams unjoined and in a flat uncontracted condition of the elastic members, the side seam and the diaper article (10) each have a longitudinal dimension. When the longitudinal dimension (LB) of either belt is greater than that (LF) of the other belt, the longitudinal dimension of the side seam equals LF. When the pant diaper article (10) has the seams unjoined and in a flat uncontracted condition of the elastic members, the longitudinal dimension between the proximal edge of the front side seam and the proximal edge of the back side seam, may be from about 40% to about 70%, preferably from about 43% to about 60% of the longitudinal dimension of the diaper article (10). By providing the pant diaper article (10) in such configuration, the absorbent assembly (20) may be appropriately placed against the wearer for wear comfort. When the distal edge of the front belt (84) and the front longitudinal end of the absorbent assembly (20) has a distance D1, and the distal edge of the back belt (86) and the back longitudinal end of the absorbent assembly (20) has a distance D2, D1 may be provided smaller than D2. By providing D1 smaller than D2, the absorbent assembly (20) may be shifted toward the front, thereby effectively receiving and absorbing fluid waste.

Referring to Figures 3A, 3B, and 4, the pant diaper article (10) of the present invention may have a chassis outer cover (422) provided on the garment facing side of the chassis backsheet (252). The chassis outer cover (422) may be any nonwoven material suitable for the first outer cover (421) mentioned above. When a chassis outer cover (422) is present, the chassis outer cover (422) may extend through the crotch region (30) but only partly in the longitudinal direction in the front and back regions (26, 28) to leave the distal parts of the front belt (84) and the back belt (86) free of the chassis outer cover (422). Namely, the longitudinal length of the chassis outer cover (422) may be longer than the distance between the front and back belts (84, 86), and shorter than the longitudinal length of the chassis backsheet (252). By such configuration, the distal parts of the front belt (84) and the back belt (86) are devoid of the chassis outer cover (422), thereby provide the overall article (10) relatively thin, and provide cost saving.

### Other components

The absorbent assembly (20) of the present invention may further comprise components that improve leakage prevention, wearability, fit, or aesthetic aspects of the resulting diaper article (10). As is typically provided for diaper articles with a single absorbent body, the chassis backsheet (252) and/or the chassis outer cover (422) may be provided with artwork which may be visible from the garment facing side of the diaper article (10). Such artwork may be coordinated with artwork provided on the fastening means, for example the elastic belts, for forming a pant article.
The first absorbent body (38) and the second absorbent body (39) may be provided with different aesthetics discernible from the topsheet side so that the user may distinguish between the first and second absorbent body (38, 39), and/or for appealing the existence of multiple absorbent bodies. For example, the first and second topsheets (241, 242) may be provided with different topography such as bondings, embossings, and openings. For example, the adhesive for bonding the first or second topsheet (241, 242) with layers directly underneath may be provided in a color and a pattern, wherein the color and/or pattern provided in the first and second absorbent body (38, 39) may be different.

Referring to Figure 1B, the garment facing side of the first absorbent body (38), namely the first outer cover (421), may be provided with a Signal Zone (SZ) which includes an indicia for intuitively guiding the user where the first absorbent body (38) may be pinched for effectively starting the removing action of the first absorbent body (38) from the remainder of the diaper article (10). The indicia may be a color and/or pattern distinguishable from the remainder of the first outer cover (421), a material and/or topography distinguishable from the remainder of the first outer cover (421), wording such as "open", "start peel" and/or combined with signals such as arrows.

### Leg Tensile Measurements

This measurement method is used for providing the following values according to the present invention: Whole Longitudinal Leg Tensile, Whole Transverse Leg Tensile, Removed Longitudinal Leg Tensile, and Removed Transverse Leg Tensile.

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the specimens tested will be between 10 and 90% of capacity of the load cell used. The instrument is calibrated according to the manufacturer's instructions.

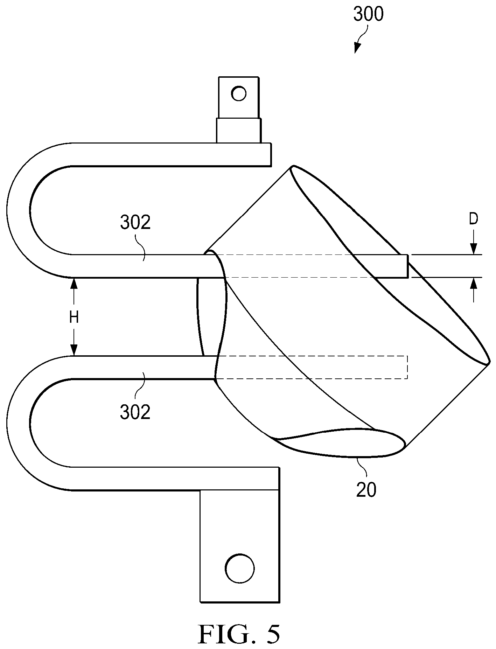

The tensile tester is fitted with hanger-type specimen holding fixtures such as in Figure 5. Each fixture comprises a rigid linear rubber-coated horizontal bar section to prevent specimen slippage during testing. The outer bar diameter (including the rubber coating) of the horizontal bar sections is 10.0 mm. The central axes of the horizontal bar sections are configured to remain parallel and in the same vertical plane throughout the test procedure.

The gauge circumference is determined by the following equation:$Gauge Circumference = 2 \times \left(H + D + πD/2\right)$

Where H is the vertical gap between the horizontal bar sections, and D is the outer diameter of the bar. All testing is performed in a room maintained at 23 ± 2 °C and 50 ± 5 % relative humidity. The instrument is set up to go through the following steps:

| | |
|---|---|
| Crosshead Speed | 254.0 mm/min |
| Data Acquisition Rate | 50 Hz |
| Final Load Point | 6N |
| Hold Time | 0 |
| Number of Cycles | 1 |

For measuring the Whole Longitudinal/Transverse Leg Tensile, the finished product should be used as test specimen. For measuring Removed Longitudinal/Transverse Leg Tensile, the first absorbent body should be peeled off from finished product, then the rest of the diaper article should be used as test specimen.

For measuring the Whole/Removed Longitudinal Leg Tensile, the specimen is inserted onto the upper horizontal bar section from one leg opening while positioning the article with the crotch side down, so that the bar passes through the one leg opening and waist opening of the article as in Figure 5. The crosshead is raised until the specimen hangs above the lower bar and does not touch lower bar. The load cell is reset to zero and the crosshead is lowered to enable the lower bar to be inserted through the leg opening without stretching the article. The specimen is adjusted so that that the side seam is on the same plane as the upper and lower bars (302). The crosshead is raised slowly while the specimen is held in place by hand as necessary until the force is between 0.05 and 0.1 N, while taking care not to add any unnecessary force. The gauge circumference at this point is the Initial Gauge Circumference. The test is initiated and the crosshead moves up at 254 mm/min until a force of 6 N is attained, then the crosshead immediately returns to the Initial Gauge Circumference at the same speed. The tensile force when the distance between the bars reaches 80.5 mm when the crosshead is returning is recorded and defined as the Whole/Removed Longitudinal Leg Tensile.

For measuring the Whole/Removed Transverse Leg Tensile, the specimen is inserted onto the upper horizontal bar section from one leg opening as in Figure 5, then specimen is then rotated 90 degrees so that the side seam is perpendicular to the hangers. The crosshead is raised until the specimen hangs above the lower bar and does not touch lower bar. The load cell is reset to zero and the crosshead is lowered to enable the lower bar to be inserted through the leg opening without stretching the article. The crosshead is raised slowly while the specimen is held in place by hand as necessary until the force is between 0.05 and 0.1 N, while taking care not to add any unnecessary force. The gauge circumference at this point is the Initial Gauge Circumference. The test is initiated and the crosshead moves up at 254 mm/min until a force of 6 N is attained, then the crosshead immediately returns to the Initial Gauge Circumference at the same speed. The tensile force when the distance between the bars reaches 60 mm when the crosshead is returning is recorded and defined as the Whole/Removed Transverse Leg Tensile.

Each of the Whole Longitudinal Leg Tensile, Whole Transverse Leg Tensile, Removed Longitudinal Leg Tensile, and Removed Transverse Leg Tensile are measured as described above for both left and right leg openings for 5 specimens, and are averaged and reported to the nearest 0.01 N.

### Peeling Strength Measurement

### 1. Preparation of finished product specimen

Specimen for the measurements hereinbelow is obtained from 3 finished diaper article samples. When the article is a pant, peel apart the side seam of the pant. The article is then stretched in the longitudinal direction such that any longitudinally extending elastic bodies, such as cuff elastics, are fully stretched, and then the article is attached on an appropriate board having at least an area of the fully stretched article via any attachment means, such as tape, adhesive, hooks, or the like. Identify which of the front or back side ("peeling side") the first absorbent body is intended to be peeled off from the remainder of the article. Cut by scissors along the width direction (transverse direction) at 10cm from the longitudinal edge of the first absorbent body on the peeling side by scissors. The portion exposed by such cutting is the measurement edge.

### 2. MTS test setting and measuring

As equipment, MTS Criterion Model 42 running TW Elite 4.3.1.375 software with standard tensiometer or equivalent is used. All testing is performed in a room maintained at 23 ± 2 °C and 50 ± 5 % relative humidity.

The initial distance between the upper grip and lower grip is set to be 20 mm. Grips having a transverse dimension greater than the width of the Temporary Bonding on the left and right sides are selected, such that the specimen is peeled at the same horizontal level simultaneously. The measurement edge of the specimen is gripped by the grips such that the upper grip is clamping the first absorbent body, and the lower grip is clamping the remainder of the article. The specimen is set such that the transverse direction of the specimen matches the horizontal direction of the equipment. A constant rate of extension of 500mm/min is applied. The elongation measurement is taken from the point where the program starts. The upper grip starts moving up, while the lower grip remains static, resulting in the first absorbent body's peeling off. The program stops until the upper grip moves 200 mm. The force (N) is continuously measured by the machine at a sampling rate of 50 Hz.

The Peak Force is the maximum force value observed during the measurement process. The Average Force is the average value observed between 80mm and 160mm extension. For both values, measurements from 3 specimens are obtained and averaged up to 0.01N preciseness.

### Folded Thickness Measurement

### 1. Preparation of finished product specimen

Specimen for the measurements hereinbelow is obtained for 3 finished diaper articles. The folded longitudinal center of the article is called the "bottom".

### 2. Folded thickness measurement

As equipment, Ono Sokki Closed Shaft foot caliper with 24.13 mm diameter foot exerting 0.689 kPa on Ames stand with Caliper Gauge GS-503 or equivalent; Digital Readout DG-3610 Measure all or equivalent are used. All testing is performed in a room maintained at 23 ± 2 °C and 50 ± 5 % relative humidity.

The specimens are obtained immediately after they are removed from a freshly opened package, and subject to the following measurement, such that measurement may be completed in 5 minutes from opening the package:
(1) Lift the caliper foot carefully by hand, lay the specimen flat on the caliper stand and place the caliper foot on the specimen such that the caliper foot is placed 5mm away from the bottom, and at the transverse center. Wait approximately 5 seconds and take the reading. The reading is the thickness of the bottom of the article.
(2) Lift the caliper foot carefully by hand, lay the specimen flat on the caliper stand and place the caliper foot on the specimen such that the caliper foot is placed 50mm away from the bottom, and at the transverse center. Wait approximately 5 seconds and take the reading. The reading is the thickness of the bottom of the article.
(3) The value obtained by steps (1) and (2) are averaged.

The Folded Thickness is the average of the value obtained in (3) from 3 samples averaged up to 0.1mm preciseness.

### EXAMPLES

Examples 1-2 are Size 4 (L-size) belt-type pant diaper articles having 2 absorbent bodies and with configuration of Figures 3A and 3B respectively, and the dimensions of Table 1 below. Examples 1-2 have Peak Force, Average Force, Whole Longitudinal Leg Tensile, Whole Transverse Leg Tensile, Removed Longitudinal Leg Tensile, and Removed Transverse Leg Tensile as in Table 1 below.

**Table 1**

| Venue | Examples 1 | Example 2 |
|---|---|---|
| Cuff Structure | Figure 3A | Figure 3B |
| T1 (mm) | 150 | 150 |
| T2 (mm) | 150 | 150 |
| T3 (mm) | 140 | 140 |
| T4 (mm) | 198 | 160 |
| TC1 (mm) | 142 | 132 |
| TC2 (mm) | 157 | 153 |
| L1 (mm) | 370 | 370 |
| L2 (mm) | 370 | 370 |
| L3 (mm) | 403 | 403 |
| L4 (mm) | 403 | 403 |
| LC1 (mm) | 311 | 311 |
| LC2 (mm) | 215 | 215 |
| H1 (mm) | 10 | 30 |
| H2 (mm) | 27 | 17 |
| Whole Longitudinal Leg Tensile (N) | 2.65 | 1.53 |
| Whole Transverse Leg Tensile (N) | 0.99 | 0.9 |
| Removed Longitudinal Leg Tensile (N) | 1.75 | 0.96 |
| Removed Transverse Leg Tensile (N) | 0.66 | 0.45 |
| Removed Longitudinal Leg Tensile/Whole Longitudinal Leg Tensile | 66% | 53% |
| Removed Transverse Leg Tensile/Whole Transverse Leg Tensile | 67% | 50% |
| Peak Force (N) of Temporary Bond | 7.17 | 12.71 |
| Average Force (N) of Temporary Bond | 0.67 | 0.5 |
| Folded Thickness (mm) | 11.0 | 11.0 |

The pant diaper article of Examples 1-2 provide two layers of absorbent bodies for two usages wherein the absorbent body for second usage remains substantially intact during the first usage. The Temporary Bonds of pant diaper article of Examples 1-2 endure the forces created during the first usage by the wearer, and are capable of being broken without destroying other components of the pant diaper article. The pant diaper article of Examples 1-2 are comfortable to wear in the first usage as well as the second usage. The pant diaper article of Examples 1-2 offers the possibility of removing the first absorbent body from the remainder of the article while the wearer is standing, which enables easier and faster diaper change.

### Consumer acceptance

9 panelists in Frankfurt, Germany who were users of similar price range products as the target consumers having babies wearing Size 4 diapers and aged 15-24 months were recruited. They were asked to use the products they used most often (hereinafter "benchmark") for 2 days, and then to use pant diaper article samples of Example 2 (hereinafter "Example 2") for 2 days, and answer a set of questions.

Table 2 shows results of the overall leakage incidence. Table 3 shows the percentages of postures the babies took for changing diaper. The percentages do not add up to 100%, as there were other postures taken. Table 4 shows the average wear time during daytime.

**Table 2**

| Sample | Benchmark | Example 2 |
|---|---|---|
| Total number of diaper change | 95 | 94 |
| Overall Leakage incidence (%) | 4.2 | 6.4 |

**Table 3**

| | Benchmark | First usage | Second usage |
|---|---|---|---|
| Total number of diaper change | 95 | 54 | 40 |
| Posture for changing diaper - standing (%) | 6 | 22 (*1) | 28 (*1) |
| Posture for changing diaper - lying on back (%) | 93 | 68 (*2) | 62 (*2) |

| | | | |
|---|---|---|---|
| (*1) Statistically significantly greater than benchmark (*2) Statistically significantly smaller than benchmark | | | |

**Table 4**

| | Benchmark | First usage | Second usage |
|---|---|---|---|
| Diaper change number during day | 79 | 48 | 38 |
| Wear time (min) | 189 | 208 | 202 |

According to the data of Table 2, the pant diaper article of Example 2 provided parity leakage incidence compared to benchmark, indicating that similar containment and leakage prevention performance was provided compared to benchmark. According to the data of Table 3, the pant diaper article of Example 2 enabled significantly higher percentage of diaper change in the standing posture compared to benchmark, indicating easier diaper change compared to benchmark. According to the data of Table 4, each usage of the pant diaper article of Example 2 provided parity wear time as that of the benchmark, indicating that the pant diaper article of Example 2 provided roughly 2 times wear time as that of the benchmark.

## Claims

1. A pant type absorbent article (10) comprising an absorbent assembly (20) and an elastic belt (40), the absorbent assembly comprising:
1) a first absorbent body (38) comprising:
a) a first water permeable topsheet (241);
b) a first water impermeable backsheet (251);
c) a first absorbent core (621) disposed between the first topsheet and the first backsheet;
d) a first outer cover (421) disposed on the garment facing side of the first backsheet;
e) a pair of first backsheet fold overs (261) extending from the transverse ends of the first backsheet and folded to the wearer facing side;
f) a pair of first outer cover fold overs (43) extending from the transverse ends of the first outer cover and folded to the wearer facing side to sandwich the first backsheet fold over (261), the transverse end of the first outer cover fold over attached to the wearer facing side of the first topsheet, wherein the first backsheet fold over (261) and the first outer cover fold over (43) form a pair of first cuffs (36), each first cuff comprising a longitudinally extending base and a longitudinally extending free edge; and
g) a first cuff elastic element (37) disposed at the free edge of each first cuff;
2) a second absorbent body (39) disposed on the garment facing side of the first absorbent body, comprising:
a) a second water permeable topsheet (242); and
b) a second absorbent core (622);
3) a chassis (100) disposed on the garment facing side of the second absorbent body, the chassis comprising:
a) a water impermeable chassis backsheet (252) having a greater transverse dimension than the first absorbent body;
b) a pair of chassis cuffs (34) formed by a cuff material and disposed on the chassis backsheet, the chassis cuffs each comprising a longitudinally extending base (34B) and a longitudinally extending free edge (34E); wherein the transverse dimension of the spacing between the bases of the pair of chassis cuffs TC2 are greater than the transverse dimension of the spacing between the bases of the pair of first cuffs TC1;
c) a chassis cuff elastic element (35) disposed at the free edge of each chassis cuff;
wherein the absorbent article exhibits a Whole Longitudinal Leg Tensile and a Whole Transverse Leg Tensile, the absorbent article devoid of the first absorbent body exhibits a Removed Longitudinal Leg Tensile and a Removed Transverse Leg Tensile, wherein the Removed Longitudinal Leg Tensile is at least about 50% of the Whole Longitudinal Leg Tensile, and the Removed Transverse Leg Tensile is at least about 50% of the Whole Transverse Leg Tensile, according to the Leg Tensile Measurements described herein.

2. The absorbent article of Claim 1, wherein the difference between the Removed Longitudinal Leg Tensile and the Whole Longitudinal Leg Tensile is less than about 1.5N, and the difference between the Removed Transverse Leg Tensile and the Whole Transverse Leg Tensile is less than about 1.5N.

3. The absorbent article of Claim 1 or 2, wherein the Whole Longitudinal Leg Tensile is from about 1N to about 3.5N, preferably from about 1.3N to about 3.3N, and the Whole Transverse Leg Tensile is from about 0.5N to about 2N, preferably from about 0.7N to about 1.3N.

4. The absorbent article of any of the preceding claims, wherein the Removed Longitudinal Leg Tensile is from about 0.5N to about 3N, preferably from about 0.8N to about 2.3N, and the Removed Transverse Leg Tensile is from about 0.2N to about 1N, preferably from about 0.3N to about 0.8N.

5. The absorbent article of any of the preceding claims, wherein the first cuff elastic element and the chassis cuff elastic element are a plurality of elastic strands, respectively, wherein there are more number of chassis cuff elastic strands than first cuff elastic strands.

6. The absorbent article of any of the preceding claims, wherein
the first cuff has a first cuff height H1 which is the transverse dimension between the base and the free edge;
the chassis cuff has a chassis cuff height H2 which is the transverse dimension between the base and the free edge, wherein H2 is greater than H1,
wherein the chassis further comprises a pair of outer cuffs disposed transversely outboard of the pair of chassis cuffs, respectively, each outer cuff formed by the chassis backsheet, the cuff material, and an outer cuff elastic element sandwiched therebetween.

7. The absorbent article of Claim 6, wherein the chassis further comprises a chassis outer cover on the garment facing side of the chassis backsheet, wherein the transverse dimension of the chassis backsheet matches that of the chassis outer cover.

8. The absorbent article of any of Claims 1-5, wherein an edge elastic element is disposed adjacent each base of the chassis cuffs,
wherein the first cuff has a first cuff height H1 which is the transverse dimension between the base and the free edge;
the chassis cuff has a chassis cuff height H2 which is the transverse dimension between the base and the free edge, wherein H2 is smaller than H1.

9. The absorbent article of Claim 8, wherein the chassis further comprises a pair of chassis backsheet fold overs extending from the transverse ends of the chassis backsheet and folded to the wearer facing side, wherein the cuff material sandwiches the chassis backsheet fold over, the chassis cuff elastic element, and edge elastic element to form the chassis cuffs.

10. The absorbent article of Claim 8 or 9, wherein the chassis further comprises a chassis outer cover on the garment facing side of the chassis backsheet, wherein the base of the chassis cuff is disposed adjacent the transverse ends of the chassis outer cover.

11. The absorbent article of any of the preceding claims, wherein when the first backsheet has a transverse dimension T1, T1 is greater than TC1.

12. The absorbent article of any of the preceding claims, wherein the first outer cover has a transverse dimension T2, TC2 is greater than T2.

13. The absorbent article of any of the preceding claims, wherein the first cuff elastic element has an effective longitudinal elasticity dimension of LC1 and the chassis cuff elastic element has an effective longitudinal elasticity of LC2, LC1 is greater than LC2.

## Patentansprüche

1. Höschenartiger Absorptionsartikel (10), umfassend eine Absorptionsgruppe (20) und einen Gummibandbund (40), die Absorptionsgruppe umfassend:
1) einen ersten Absorptionskörper (38), umfassend:
a) eine erste wasserdurchlässige Oberschicht (241);
b) eine erste wasserundurchlässige Unterschicht (251);
c) einen Absorptionskern (621), der zwischen der ersten Oberschicht und der ersten Unterschicht eingerichtet ist;
d) einen ersten Außenmantel (421), der auf der bekleidungsseitigen Seite der ersten Unterschicht eingerichtet ist;
e) ein Paar Umfaltungen (261) der ersten Unterschicht, die sich von den Querenden der ersten Unterschicht erstrecken und zu der trägerseitigen Seite gefaltet sind;
f) ein Paar Umfaltungen (43) des ersten Außenmantels, die sich von den Querenden des ersten Außenmantels erstrecken und zu der trägerseitigen Seite gefaltet sind, um die Umfaltung (261) der ersten Unterschicht in Sandwichform anzuordnen, wobei das Querende der Umfaltung des ersten Außenmantels an der trägerseitigen Seite der ersten Oberschicht angebracht ist, wobei die Umfaltung (261) der ersten Unterschicht und die Umfaltung (43) des ersten Außenmantels ein Paar erster Bündchen (36) bilden, jedes erste Bündchen umfassend eine sich längs erstreckende Basis und einen sich längs erstreckenden freien Rand; und
g) ein Gummibandelement (37) des ersten Bündchens, das an dem freien Rand jedes ersten Bündchens eingerichtet ist;
2) einen zweiten Absorptionskörper (39), der auf der bekleidungsseitigen Seite des ersten Absorptionskörpers eingerichtet ist, umfassend:
a) eine zweite wasserdurchlässige Oberschicht (242); und
b) einen zweiten Absorptionskern (622);
3) eine Grundeinheit (100), die auf der bekleidungsseitigen Seite des zweiten Absorptionskörpers eingerichtet ist, die Grundeinheit umfassend:
a) eine wasserundurchlässige Grundeinheitsunterschicht (252), die eine größere Querabmessung als der erste Absorptionskörper aufweist;
b) ein Paar Grundeinheitsbündchen (34), die durch ein Bündchenmaterial gebildet und auf der Grundeinheitsunterschicht eingerichtet sind, die Grundeinheitsbündchen jeweils umfassend eine sich längs erstreckende Basis (34B) und einen sich längs erstreckenden freien Rand (34E); wobei die Querabmessung des Abstands zwischen den Basen des Paars Grundeinheitsbündchen TC2 größer als die Querabmessung des Abstands zwischen den Basen des Paars erster Bündchen TC1 ist;
c) ein Grundeinheitsbündchengummibandelement (35), das an dem freien Rand jedes Grundeinheitsbündchens eingerichtet ist;
wobei der Absorptionsartikel eine ganze Längsbeinzugfestigkeit und eine ganze Querbeinzugfestigkeit vorweist, der Absorptionsartikel ohne den ersten Absorptionskörper eine entfernte Längsbeinzugfestigkeit und eine entfernte Querbeinzugfestigkeit vorweist, wobei die entfernte Längsbeinzugfestigkeit wenigstens etwa 50 % der ganzen Längsbeinzugfestigkeit beträgt, und die entfernte Querbeinzugfestigkeit wenigstens etwa 50 % der ganzen Querbeinzugfestigkeit beträgt, gemäß den hierin beschriebenen Beinzugfestigkeitsmessungen.

2. Absorptionsartikel nach Anspruch 1, wobei die Differenz zwischen der entfernten Längsbeinzugfestigkeit und der ganzen Längsbeinzugfestigkeit weniger als etwa 1,5 N beträgt, und die Differenz zwischen der entfernten Querbeinzugfestigkeit und der ganzen Querbeinzugfestigkeit weniger als etwa 1,5 N beträgt.

3. Absorptionsartikel nach Anspruch 1 oder 2, wobei die ganze Längsbeinzugfestigkeit von etwa 1 N bis etwa 3,5 N, vorzugsweise von etwa 1,3 N bis etwa 3,3 N, beträgt, und die ganze Querbeinzugfestigkeit von etwa 0,5 N bis etwa 2 N, vorzugsweise von etwa 0,7 N bis etwa 1,3 N, beträgt.

4. Absorptionsartikel nach einem der vorstehenden Ansprüche, wobei die entfernte Längsbeinzugfestigkeit von etwa 0,5 N bis etwa 3 N, vorzugsweise von etwa 0,8 N bis etwa 2,3 N, beträgt, und die entfernte Querbeinzugfestigkeit von etwa 0,2 N bis etwa 1 N, vorzugsweise von etwa 0,3 N bis etwa 0,8 N, beträgt.

5. Absorptionsartikel nach einem der vorstehenden Ansprüche, wobei das Gummibandelement des ersten Bündchens beziehungsweise das Grundeinheitsbündchengummibandelement eine Vielzahl von Gummibandsträngen ist, wobei eine größere Anzahl von Grundeinheitsbündchengummibandsträngen als Gummibandstränge des ersten Bündchens vorhanden ist.

6. Absorptionsartikel nach einem der vorstehenden Ansprüche, wobei
das erste Bündchen eine Höhe H1 des ersten Bündchens aufweist, die die Querabmessung zwischen der Basis und dem freien Rand beträgt;
das Grundeinheitsbündchen eine Grundeinheitsbündchenhöhe H2 aufweist, die die Querabmessung zwischen der Basis und dem freien Rand beträgt, wobei H2 größer als H1 ist,
wobei die Grundeinheit ferner ein Paar Außenbündchen umfasst, die jeweils querverlaufend außerhalb des Paars Grundeinheitsbündchen eingerichtet sind, wobei jedes Außenbündchen durch die Grundeinheitsunterschicht, das Bündchenmaterial und ein Außenbündchengummibandelement, das in Sandwichform dazwischen angeordnet ist, gebildet ist.

7. Absorptionsartikel nach Anspruch 6, wobei die Grundeinheit ferner einen Grundeinheitsaußenmantel auf der bekleidungsseitigen Seite der Grundeinheitsunterschicht umfasst, wobei die Querabmessung der Grundeinheitsunterschicht mit der des Grundeinheitsaußenmantels übereinstimmt.

8. Absorptionsartikel nach einem der Ansprüche 1 bis 5, wobei ein Randgummibandelement benachbart zu jeder Basis der Grundeinheitsbündchen eingerichtet ist,
wobei das erste Bündchen eine Höhe H1 des ersten Bündchens aufweist, die die Querabmessung zwischen der Basis und dem freien Rand beträgt;
das Grundeinheitsbündchen eine Grundeinheitsbündchenhöhe H2 aufweist, die die Querabmessung zwischen der Basis und dem freien Rand beträgt, wobei H2 kleiner als H1 ist.

9. Absorptionsartikel nach Anspruch 8, wobei die Grundeinheit ferner ein Paar Grundeinheitsunterschichtumfaltungen umfasst, die sich von den Querenden der Grundeinheitsunterschicht erstrecken und zu der trägerseitigen Seite gefaltet sind, wobei das Bündchenmaterial die Grundeinheitsunterschichtumfaltung, das Grundeinheitsbündchengummibandelement und das Randgummibandelement in Sandwichform anordnet, um das Grundeinheitsbündchen zu bilden.

10. Absorptionsartikel nach Anspruch 8 oder 9, wobei die Grundeinheit ferner einen Grundeinheitsaußenmantel auf der bekleidungsseitigen Seite der Grundeinheitsunterschicht umfasst, wobei die Basis des Grundeinheitsbündchens benachbart zu den Querenden des Grundeinheitsaußenmantels eingerichtet ist.

11. Absorptionsartikel nach einem der vorstehenden Ansprüche, wobei, wenn die erste Unterschicht eine Querabmessung T1 aufweist, T1 größer als TC1 ist.

12. Absorptionsartikel nach einem der vorstehenden Ansprüche, wobei der erste Außenmantel eine Querabmessung T2 aufweist, wobei TC2 größer als T2 ist.

13. Absorptionsartikel nach einem der vorstehenden Ansprüche, wobei das Gummibandelement des ersten Bündchens eine effektive Längselastizitätsabmessung von LC1 aufweist und das Grundeinheitsbündchengummibandelement eine effektive Längselastizität von LC2 aufweist, wobei LC1 größer als LC2 ist.

## Revendications

1. Article absorbant de type culotte (10) comprenant un ensemble absorbant (20) et une ceinture élastique (40), l'ensemble absorbant comprenant :
1) un premier corps absorbant (38) comprenant :
a) une première feuille de dessus (241) perméable à l'eau ;
b) une première feuille de fond (251) imperméable à l'eau ;
c) une première âme absorbante (621) disposée entre la première feuille de dessus et la première feuille de fond ;
d) une première couverture externe (421) disposée sur le côté face au vêtement de la première feuille de fond ;
e) une paire de premiers rabats de feuille de fond (261) s'étendant à partir des extrémités transversales de la première feuille de fond et pliés vers le côté face au porteur ;
f) une paire de premiers rabats de couverture externe (43) s'étendant à partir des extrémités transversales de la première couverture externe et pliés vers le côté face au porteur pour prendre en sandwich le premier rabat de feuille de fond (261), l'extrémité transversale du premier rabat de couverture externe étant fixée au côté face au porteur de la première feuille de dessus, dans lequel le premier rabat de feuille de fond (261) et le premier rabat de couverture externe (43) forment une paire de premiers revers (36), chaque premier revers comprenant une base s'étendant longitudinalement et un bord libre s'étendant longitudinalement ; et
g) un premier élément élastique de revers (37) disposé au niveau du bord libre de chaque premier revers ;
2) un second corps absorbant (39) disposé sur le côté face au vêtement du premier corps absorbant, comprenant :
a) une seconde feuille de dessus (242) perméable à l'eau ; et
b) une seconde âme absorbante (622) ;
3) un châssis (100) disposé sur le côté face au vêtement du second corps absorbant, le châssis comprenant :
a) une feuille de fond de châssis (252) imperméable à l'eau ayant une dimension transversale supérieure au premier corps absorbant ;
b) une paire de revers de châssis (34) formés par un matériau de revers et disposés sur la feuille de fond de châssis, les revers de châssis comprenant chacun une base (34B) s'étendant longitudinalement et un bord libre (34E) s'étendant longitudinalement ; dans lequel la dimension transversale de l'espacement entre les bases de la paire de revers de châssis TC2 est supérieure à la dimension transversale de l'espacement entre les bases de la paire de premiers revers TC1 ;
c) un élément élastique de revers de châssis (35) disposé au niveau du bord libre de chaque revers de châssis ;
dans lequel l'article absorbant présente un étirement de jambe longitudinal total et un étirement de jambe transversal total, l'article absorbant dépourvu du premier corps absorbant présente un étirement de jambe longitudinal réduit et un étirement de jambe transversal réduit, dans lequel l'étirement de jambe longitudinal réduit est d'au moins environ 50 % de l'étirement de jambe longitudinal total, et l'étirement de jambe transversal réduit est d'au moins environ 50 % de l'étirement de jambe transversal total, selon les mesures d'étirement de jambe décrites ici.

2. Article absorbant selon la revendication 1, dans lequel la différence entre l'étirement de jambe longitudinal réduit et l'étirement de jambe longitudinal total est inférieure à environ 1,5 N, et la différence entre l'étirement de jambe transversal réduit et l'étirement de jambe transversal total est inférieure à environ 1,5 N.

3. Article absorbant selon la revendication 1 ou 2, dans lequel l'étirement de jambe longitudinal total est d'environ 1 N à environ 3,5 N, de préférence d'environ 1,3 N à environ 3,3 N, et l'étirement de jambe transversal total est d'environ 0,5 N à environ 2 N, de préférence d'environ 0,7 N à environ 1,3 N.

4. Article absorbant selon l'une quelconque des revendications précédentes, dans lequel l'étirement de jambe longitudinal réduit est d'environ 0,5 N à environ 3 N, de préférence d'environ 0,8 N à environ 2,3 N, et l'étirement de jambe transversal réduit est d'environ 0,2 N à environ 1 N, de préférence d'environ 0,3 N à environ 0,8 N.

5. Article absorbant selon l'une quelconque des revendications précédentes, dans lequel le premier élément élastique de revers et l'élément élastique de revers de châssis sont une pluralité de brins élastiques, respectivement, dans lequel il y a plus de brins élastiques de revers de châssis en nombre que de premiers brins élastiques de revers.

6. Article absorbant selon l'une quelconque des revendications précédentes, dans lequel
le premier revers a une hauteur de premier revers H1 qui est la dimension transversale entre la base et le bord libre ;
le revers de châssis a une hauteur de revers de châssis H2 qui est la dimension transversale entre la base et le bord libre, dans lequel H2 est supérieure à H1,
dans lequel le châssis comprend en outre une paire de revers externes disposés transversalement à l'extérieur de la paire de revers de châssis, respectivement, chaque revers externe étant formé par la feuille de fond de châssis, le matériau de revers, et un élément élastique de revers externe pris en sandwich entre eux.

7. Article absorbant selon la revendication 6, dans lequel le châssis comprend en outre une couverture externe de châssis sur le côté face au vêtement de la feuille de fond de châssis, dans lequel la dimension transversale de la feuille de fond de châssis concorde avec celle de la couverture externe de châssis.

8. Article absorbant selon l'une quelconque des revendications 1 à 5, dans lequel un élément élastique de bord est disposé adjacent à chaque base des revers de châssis,
dans lequel le premier revers a une hauteur de premier revers H1 qui est la dimension transversale entre la base et le bord libre ;
le revers de châssis a une hauteur de revers de châssis H2 qui est la dimension transversale entre la base et le bord libre, dans lequel H2 est inférieure à H1.

9. Article absorbant selon la revendication 8, dans lequel le châssis comprend en outre une paire de rabats de feuille de fond de châssis s'étendant à partir des extrémités transversales de la feuille de fond de châssis et pliés jusqu'au côté face au porteur, dans lequel le matériau de revers prend en sandwich le rabat de feuille de fond de châssis, l'élément élastique de revers de châssis, et l'élément élastique de bord pour former les revers de châssis.

10. Article absorbant selon la revendication 8 ou 9, dans lequel le châssis comprend en outre une couverture externe de châssis sur le côté face au vêtement de la feuille de fond de châssis, dans lequel la base du revers de châssis est disposée adjacente aux extrémités transversales de la couverture externe de châssis.

11. Article absorbant selon l'une quelconque des revendications précédentes, dans lequel lorsque la première feuille de fond a une dimension transversale T1, T1 est supérieure à TC1.

12. Article absorbant selon l'une quelconque des revendications précédentes, dans lequel la première couverture externe a une dimension transversale T2, TC2 est supérieure à T2.

13. Article absorbant selon l'une quelconque des revendications précédentes, dans lequel le premier élément élastique de revers a une dimension d'élasticité longitudinale efficace de LC1 et l'élément élastique de revers de châssis a une élasticité longitudinale efficace de LC2, LC1 est supérieure à LC2.
